# EUROPEAN PATENT SPECIFICATION

(11) **EP 2 035 813 B1**
(45) Date of publication and mention of the grant of the patent: **06.06.2012**
(21) Application number: 07786732.3
(22) Date of filing: 13.06.2007
(51) Int. Cl.: G01N 21/78, G01N 21/45, G01N 33/12

(54) **OPTICAL SENSOR AND METHOD FOR INDICATING THE AGE OR QUALITY OF A NATURAL PRODUCT**
OPTISCHER SENSOR UND VERFAHREN ZUR ANZEIGE DES ALTERS ODER DER QUALITÄT EINES NATURPRODUKTS
DISPOSITIF POUR ANALYSER L'ÂGE ET/OU LA QUALITÉ D'UN PRODUIT NATUREL (CAPTEUR DE FRAÎCHEUR INTÉGRÉ)

(30) Priority: 16.06.2006 AT 10262006
(43) Date of publication of application: 18.03.2009
(73) Proprietor: Sony DADC Austria AG, 5081 Anif (AT)
(72) Inventor: BAUER, Maria, 7020 Salzburg (AT); PITTNER, Fritz, 1230 Wien (AT)
(74) Representative: Bühler, Dirk
(86) International application number: PCT/EP2007/055812
(87) International publication number: WO 2007/144367

(56) References cited:
- WO-A-2004/025254
- WO-A-2005/111588
- JP-A- 2004 344 056
- US-A- 5 611 998
- US-A1- 2004 062 682
- US-A1- 2005 249 899
- AUSSENEGG F R ET AL: "The metal island coated swelling polymer over mirror system (MICSPOMS): A new principle for measuring ionic strength" SENSORS AND ACTUATORS B, ELSEVIER SEQUOIA S.A., LAUSANNE, CH, vol. 29, no. 1, October 1995 (1995-10), pages 204-209, XP004000873 ISSN: 0925-4005

## Description

### FIELD OF THE INVENTION

The present invention relates to the field of optical sensors for analyzing the age and/or quality of a natural product comprising foods and cosmetic products.

In this context, the invention describes a sensor, in which an analyte-sensitive layer is embedded in an optical thin layer assembly. The invention also describes the preparation and use of the sensor.

### BACKGROUND OF THE INVENTION

Aging processes leading to the spoilage of foods are often caused by microorganisms. Examples for the spoilage of foods caused by microorganisms comprise: the spoilage of fish and meat caused by the infestation with e.g. *Acetinobacter, Moraxella, Pseudomonas species, Lactobacillus species, Bacillus sp., Micrococcus, Clastridium botulinum;* the spoilage of milk products caused by the infestation with Lactic acid bacteria; the spoilage of foods containing carbohydrates such as bread caused by molds.

At present, the spoilage of such foods is detected by consumers by means of tasting such foods or by the visible decay of foods in the advanced stages of decay. The main disadvantage of this solution is that the consumer can suffer major health damage, even in case of small contamination by microorganisms. At present, foods are for this reason randomly tested in laboratories. In general, these tests rely on the proliferation of the microorganisms after several days and the visible detection of the extracted and multiplied DNA. Both methods of detection are time consuming and are not available to the consumer.

From the literature, there are known US5611998 ("Optochemical sensor and method for production") as well as the reissue derived thereof USRE37412 ("Optochemical sensor and method for production") both in the name of Aussenegg et. al., wherein a polymeric distance layer in an optical thin layer set-up is brought to swelling or shrinking by the action of a lower molecular analyte. The present invention describes an irreversible sensor, in which the sensor layer is destroyed. High molecular proteins penetrate the thin layer set-up. Also known is US6669906 ("Reinforced cluster optical sensors") in the name of Schalkhammer et. al., wherein the binding between the reflector layer and a nanoparticle is accomplished by a specific molecule. The destruction of this binding leads to an optical signal. In the literature, there are also other examples given for irreversible sensors on the basis of optical thin layers; however, a biodegradable polymer is not used as a sensor layer therein and it is not possible for the end consumer to retrieve information about a bacterial contamination of foods.

WO 2005/111588 in the name of 3M INNOVATIVE PROPERTIES COMPANY discloses colorimetric sensor films comprising a reflective layer, a polymeric detection layer, and a semi-reflective layer. Furthermore, WO 2005/111588 discloses devices comprising the colorimetric sensor films and methods of making the films and devices.

Apart from DNA, bacteria also contain specific enzymes. The relatively low activity of the enzymes secreted by bacteria to digest substrates has so far prevented the use of specific protein molecules for the detection of bacteria. The concentration of such enzymes correlates with the amount of bacteria present. Thus, the detection of enzymatic activity by the consumer may be an essential contribution to the increase in confidence of consumers to a product and in monitoring conditions of delivery and storage.

To ease quality control of foods for consumers, there is a need for a device and/or sensor that cheaply and easy visibly indicates the condition of foods. The device and/or sensor is used for the detection of specific reactions of degradation that occur during the aging of e.g. foods or cosmetic products. Such aging processes are often associated with e.g. the spoilage of foods. The quality of the product may, therefore, be checked before consumption even without any knowledge of transport and storage conditions. The device and/or sensor should give information about the microbial contamination during the total period of storage life.

### OBJECTS AND SUMMARY OF THE INVENTION

It is an object of the present invention to provide a device which can be used to analyze the age and/or quality of natural products, e.g. foods.

It is yet another object of the present invention to provide a method for preparing such a device.

It is a further object of the present invention to provide a method for analyzing the age and/or quality of a natural product with said device.

It is an object of the present invention to describe the use of said device for the analysis of the age and/or quality of a natural product.

It is a further object of the present invention to describe a sensor that makes the specific activity of microbial enzymes visible to the consumer of foods.

These and other objects of the present invention, as they will become apparent from the ensuing description, are solved by the subject matter of the independent claims. The dependent claims relate to some of the preferred embodiments of the invention.

According to one aspect of the invention, a device for analyzing the age and/or quality of a natural product is provided, comprising
(a) a reflecting layer,
(b) a nanoparticle layer,
(c) a biodegradable polymer layer positioned between said reflecting layer and said nanoparticle layer, wherein said biodegradable polymer layer is selected from the group of polymers comprising polylactic acid (PLA), poly-L-lactic acid (PLLA), PLGA, PHB and polyvinyl caprolactame (PVCL). Said device is configured in such a way that biomolecules capable of degrading said polymer layer are able to penetrate said reflecting layer and/or said nanoparticle layer in order to contact said polymer layer. Furthermore, said device is configured in such a way that a change in the thickness of said polymer layer is visible to the human eye.

In a preferred embodiment of the present invention, the reflecting and/or nanoparticle layers have a thickness of 1 to 100 nm. In a further preferred embodiment of the present invention, the biodegradable polymer layer has a thickness of 5 to 1000 nm.

In a further preferred embodiment of the invention, the reflecting layer is a mirror layer made of a metal. In yet another embodiment of the present invention, said reflecting/mirror layer is made of gold or titanium or TiAl or Inconnel. In a preferred embodiment of the present invention, the reflecting layer made of gold has a thickness of 10 to 30 nm.

In a further aspect of the present invention, the nanoparticle layer comprises a plurality of island-like structures wherein said island-like structures have a size of 5 to 50 nm in diameter. In yet another embodiment of the present invention, the nanoparticle layer is made of a metal. In still another embodiment of the present invention, the nanoparticle layer is made of gold or titanium or TiAl or Inconnel. In a preferred aspect of the present invention, the nanoparticle layer is made of gold and has a thickness of 2 to 20 nm.

Both, the reflecting and the nanoparticle layer, are in further embodiments of the invention from the group of metals or metallic conducting materials and show the optical behaviour of metal or metal like materials.

In yet another aspect of the present invention, the biodegradable polymer layer is degradable by biomolecules comprising enzymes and/or catabolic metabolites.

In another preferred embodiment of the present invention, the biodegradable polymer layer has a thickness of 100 to 500 nm.

In still another embodiment of the present invention, the device described above comprises an additional carrier layer on the reflecting layer and/or the nanoparticle layer, in each case being positioned on the side of the reflecting and/or nanoparticle layer opposite of said polymer layer.

In yet a further aspect of the present invention, the device also comprises a reference device.

In a further preferred aspect of the invention, the device comprises
(a) a reflecting layer made of gold with a thickness of 10 to 30 nm
(b) a nanoparticle layer made of gold consisting of a plurality of island-like structures wherein said island-like structures have a size of 5 to 50 nm in diameter with a thickness of 2 to 20 nm
(c) a biodegradable polymer layer with a thickness of 100 to 500 nm positioned between said reflecting layer and said nanoparticle layer
(d) a reference device.

In yet another aspect of the present invention, a method for preparing such a device is provided. This method comprises the steps of
(a) providing a reflecting layer
(b) applying a biodegradable polymer layer onto said reflecting layer
(c) applying a nanoparticle layer onto said polymer.

In yet a further object of the present invention, an additional step is added to the method for preparing a device described above. The method comprises the additional step of providing a carrier and applying said reflecting layer onto said carrier.

In still another method for preparing a device according to the invention, the method comprises the steps of
(a) providing a carrier
(b) applying a nanoparticle layer onto said carrier
(c) applying a biodegradable polymer layer onto said reflecting layer
(d) applying a reflecting layer onto said polymer layer.

In yet another aspect of the methods of the present invention, the polymer layer is applied by dip coating or film printing.

In yet another embodiment of the method of the present invention, the nanoparticle layer is applied by sputter coating, by evaporation, or by chemical reactions.

In yet another embodiment of the method of the present invention, the reflecting layer is applied by evaporation or by sputter coating or by chemical reactions, such as direct application of gold nanoparticles through a chemical reaction using the reduction of HAuCl₄.

The present invention relates in one aspect to a method for analyzing the age and/or quality of a natural product comprising foods and cosmetic products. This method comprises the following steps:
(a) providing a device as described above
(b) contacting said device with a natural product
(c) determining the colour of said device
(d) comparing the colour of said device to the colour of a reference device
(e) determining the age and/or quality of said natural product according to this comparison.

The afore described method for analyzing the age and/or quality of a natural product comprises in a further embodiment a step (step b) listed above) wherein the reflecting layer or the nanoparticle layer of said device is being contacted with said natural product in such a way that biomolecules are able to penetrate the reflecting layer or the nanoparticle layer and contact the polymer layer.

In a preferred embodiment, a device as described above is used for the analysis of the age and/or quality of a natural product comprising foods and cosmetical products.

The present invention relates in a further preferred embodiment to the use of a device as described above for the analysis of the age and/or quality of a natural product by detecting microorganisms present in the natural product.

The present invention relates in a further preferred embodiment to the use of a device as described above for the analysis of the age and/or quality of a natural product by detecting enzymes of microorganisms and/or of the natural product and/or catabolic metabolites via the degradation of said biodegradable polymer by said enzymes and/or catabolic metabolites.

### DESCRIPTION OF THE FIGURES

Fig1:
   **Schematic view of a sensor platelet with integrated reference field.** The figure shows a schematic cross section of a sensor platelet with the following layers:
   1: carrier
   2: reflector
   3: biodegradable polymer layer
   4: mirror
Fig2:
   **Picture of a sensor platelet which has been incubated with different concentrations of esterase.** This figure shows a picture (**a**) and schematic view (**b**) of a sensor platelet, which has been incubated with different concentrations of esterase.
   5: degradation by esterase of a concentration of 10 mg/ml after 2 h
   6: degradation by esterase of a concentration of 5 mg/ml after 2 h
   7: degradation by esterase of a concentration of 2.5 mg/ml after 2 h
   8: incubation with buffer for 2 hours
   9: sensor platelet
Fig3:
   **Schematic view of the packaging with integrated indicator.** The figure shows a schematic view of a packaging with integrated freshness indicator according to the invention.
   10: packaging with partly opened lid
   11: sensor field
   12: reference field
Fig4:
   **Schematic view of the different layers used in a direct setup of the device and an indirect setup of the device, resp.** In the direct setup (**a**), the natural product and the device need to be separated at least partly in order to assess the colour of the device whereas in the indirect setup (*b*), the colour of the device can be assessed directly.
Fig5:
   **Colour change of a device according to the invention with a direct setup after incubation with different samples of fresh and old meat juice.** The device has been scanned in (**a**) with an additional parafilm on top to increase the quality of the scanned picture regarding the colours. In (**b**), the device of (a) has been scanned without an additional parafilm to make the degradation visible. The device has been incubated with different samples of fresh and old meat juice as described in example 4.
Fig6:
   **Colour change of a device according to the invention with an indirect setup after incubation with different samples of meat juice (a) and corresponding intensities of signals (b).** The device has been scanned without any filter in (a) and the intensities of the different signals 1 to 6 have been quantified (b). The device has been incubated with different samples of meat juice as described in example 5.
Fig7:
   **Exemplary use of a device according to the invention to analyze the age and/or quality of meat.** In the left case, both, the device according to the invention and the reference device, are shown. The device according to the invention is formed as stripe in the left picture and as square in the right picture. The reference device comprises four colours indicating the quality of the product ranging from "ok" to "harmful". The patterning of the device depicted here does not show a certain embodiment, it is rather meant to illustrate that the device may have different colours, e.g. red, yellow, blue or white.

### DETAILED DESCRIPTION OF THE INVENTION

As has been set out above, there is a need for a device which allows the analysis of the age and/or quality of a natural product by the consumer.

The present invention provides devices and methods for solving this need. While describing in detail exemplary embodiments of the present invention, definitions important for understanding the present invention are also given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±10 % and preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

The term "natural product" in the context of the present invention comprises any product which is subjected to spoilage and/or decay and, therefore, possesses a certain time frame in which it may be used according to its purpose. As set out in the background section, using an e.g. spoiled natural product such as eating spoiled food may lead to major health problems. Foods form a very large class of such natural products. This class is comprised of fish, meat, milk products, vegetables, carbohydrate-containing foods such as bread, and the like. Another class of natural products comprises cosmetic products, which are also subjected to spoilage and/or decay, e.g. in case of inadequate storage and/or delivery conditions and aging processes. The term "natural product" in the context of this invention does not define a "natural product" in a way that it has to be untreated. The natural product may be treated or untreated. Any natural product according to the invention may be treated and/or e.g. used in preparation processes, such as e.g. cooking, baking, boiling, freezing, and the like. "Natural" in the context of the invention rather implies that the product, although it may be pretreated, is still a substrate for spoilage and/or decay processes by e.g. microorganisms. The natural product may also be packaged in any way known to the person skilled in the art.

The term "age and/or quality" relates to the natural product as defined above. As mentioned before, natural products possess different time frames, in which they may be used according to their purposes. A very important aspect is the age of the product because of the correlation of contamination by e.g. microorganisms and time. The longer the incubation time, the more concentrated and severe the contamination. In case of inadequate storage and/or delivery conditions, this correlation may change and favour an even more severe contamination within a shorter time. For this reason, the natural product should always be subjected to controls even if it is not incubated for a long time. Therefore, "age" and "quality" in the context of the present invention mainly refer e.g. in case of foods to the edibility of such foods.

Therefore, the device according to the invention is used in a preferred embodiment to analyze if the cold chain of foods has been handled correctly. As set out above, most of the microorganisms responsible for the spoilage of foods preferably proliferate at 37°C. Therefore, many foods, e.g. meat, are stored and transported at temperatures below 37°C, preferably at 4°C or even frozen at -20°C to maintain an unfavourable temperature range for such microorganisms. As transport includes different storage areas maintained at such low temperatures as e.g. cold storage houses or adequate transport vehicles, the whole delivery process from the place of production to the place of offering such foods (e.g. a supermarket) is referred to as the cold chain. Therefore, the device according to the invention may be used by the consumer of the natural product to analyze if the natural product indeed has been handled according to the cold chain. Alternatively, the person involved in presenting and selling the product (e.g. an employee of a supermarket) may check at the arrival the quality of the natural product to decide whether storage and/or transport have been handled according to the cold chain and, therefore, whether the natural product may be presented to the comsumer.

The term "biomolecules" in the context of the present invention defines molecules present in or secreted from the natural product to be analysed and/or present in or secreted from any further object associated with said natural product, e.g a microorganism. Therefore, the biomolecule may derive from e.g. a food such as meat or from a microorganism associated with said meat. Preferably, such biomolecules comprise enzymes, such as phopholipases, hydrolases, pronases, proteinases (such as proteinase K), esterases of different types, lipases and the like. Biomolecules according to the present invention also comprise any molecules present in or secreted from the natural product to be analysed and/or present in or secreted from any further object associated with said natural product, e.g a microorganism, of non-enzymatic origin which are capable of degrading the biodegradable polymer layer, i.e. for example intermediates of the metabolism of such microorganisms associated with said natural product. Such non-enzymatic molecules are defined in the context of the invention as catabolic metabolites. Examples for such catabolic metabolites comprise volatile acids, volatile bases, volatile aldehydes, volatile mercaptans and sulfur compounds. Common to both (to the enzymes as well as to the catabolic metabolites) is their ability to degrade the polymer layer of the devices described herein.

The term "reflecting layer" describes a layer which is reflecting incident light. In the setup described herein, the most preferred light source is daylight or any other light source resembling daylight, e.g. light bulbs, neon light and so on. The incident light is reflected at a certain wavelength, which leads in combination with the other features of the present invention as described below to a certain colour of the device which is visible to the human eye, e.g. a red, white, blue or green colour. Most preferably, the colour is blue. The reflecting layer may also be called "mirror layer". In a preferred embodiment, this layer may be metallic or an alloy such as CrNi. In a further preferred embodiment this mirror layer may be made of gold. In another embodiment, aluminium or Inconnel is used as mirror layer. Preferably, the material used for the reflecting layer is inert to any reactions with the natural product. The reflecting layer is configured such that biomolecules as defined above are able to penetrate said layer in order to contact the polymer layer on the other side of the reflecting layer. Furthermore, the mirror layer should be stabile in biological buffers and have a very smooth surface.

The reflecting layer may be about 0.5 nm to about 500 nm thick, preferably it may be about 1 nm to about 100 nm thick, more preferably it may be about 5 nm to about 50 nm thick and even more preferably it may be about 10 to about 30 nm thick.

In the context of the invention, the term "nanoparticle layer" describes a layer made of a plurality of nanostructures.

These nanostructures may be positioned on the polymer layer as structures that are isolated from each other. These isolated structures may take various geometric forms such as squares, circles etc. The structures may, of course, also have an irregular shape. Given that the structures irrespective of their shape are isolated from each other, they may be designated as "island-like" nanostructures or "island-like structures".

In another embodiment the nanostructure layer is made from a meshwork of nanostructures, i.e. nanostructures which are connected with each other. Such structures may have a regular shape, i.e. take the form of a grid or an irregular shape, i.e. an irregular meshwork. The knots of such meshworks may take the shape of the above-described "island-like" structures.

The nanoparticle layer may also be a combination of these two embodiments, i.e. comprise partly isolated nanostructure and a meshwork of nanostructures.

In the setup of the present invention, the nanoparticle layer is translucent for incident light and for light reflected by the mirror layer.

Therefore, in preferred embodiments of the invention, the diameter of the island-like structures and/or of the knots of the meshwork of nanoparticles is smaller than the wavelength of the incident and reflected light.

In a preferred embodiment, the diameter of the island-like structures and/or the knots of the meshwork of nanostructures are in the range of about 1 nm to about 100 nm, preferably about 5 nm to 50 nm and more preferably of about 2 to about 20 nm.

In a preferred embodiment of the invention, the island-like structure and/or the meshwork of the nanoparticle layer are made of a metal. In a further preferred embodiment, this metal is gold or copper. As for the reflecting layer, the material for the nanoparticle layer is in a preferred embodiment inert to any reaction with the natural product. Furthermore, also the nanoparticle layer is configured such that biomolecules defined above are able to penetrate said layer in order to contact the layer on the other side of the reflecting layer.

The nanoparticle layer may be about 0.5 nm to about 500 nm thick, preferably it may be about 1 nm to about 100 nm thick, more preferably it may be about 2 nm to about 20 nm thick and even more preferably it may be about 3 nm to about 5 nm thick.

According to the invention, a "biodegradable polymer layer" is positioned between the reflecting and the nanoparticle layer. Said "biodegradable polymer layer" is, therefore, not directly exposed to the outside of the device, i.e. is not positioned on the surface of said device. As set out above, both, the reflecting- and the nanoparticle layers are configured such that biomolecules as defined above are able to penetrate said layers and, therefore, are able to contact the biodegradable polymer layer.

In one preferred embodiment, the "biodegradable polymer layer" may be the only layer of the layers comprising the nanoparticle layer, the reflecting layer and the biodegradable polymer layer, which is degradable by biomolecules as defined above. Said biomolecules penetrate the reflecting and/or nanoparticle layer and contact the biodegradable polymer layer but do not react with either the reflecting and/or nanoparticle layer. In a preferred embodiment of the invention, both, the reflecting and the nanoparticle layer, are, therefore, not degradable by said biomolecules.

The term "biodegradable" defines that the polymer layer of the present invention is degradable by biomolecules comprising enzymes and/or catabolic metabolites as defined above. Therefore, e.g. enzymes/catabolic metabolites secreted by microorganisms present in foods or enzymes/catabolic metabolites secreted by the natural product itself are capable of degrading said polymer layer. The material of the polymer layer is chosen from the group comprising polylactic acid (PLA), poly-L-lactic acid (PLLA), PLGA, PHB and Polyvinylcaprolactame (PVCL). In case the material of said polymer layer is degraded by enzymes and/or catabolic metabolites, this is accompanied by a change of the thickness of the polymer layer and, therefore, ultimately a change of the colour of the device.

In another preferred embodiment of the invention, the biodegradable polymer layer additionally comprises a cross-linking agent. This cross-linking agent may be a bifunctional agent, such as e.g. diisocyanat, glutardialedhyde or Desmodur (Desmodur 2460 M, Bayer). Desmodur products based on diphenylmethane diisocyanate (MDI), toluene diisocyanate (TDI), hexamethylene diisocyanate (HDI) and isophorone diisocyanate (IPDI) may also be used.

In still another preferred embodiment of the invention, the biodegradable polymer layer additionally comprises a solvent. This solvent may be selected from the group comprising chloroform, chloroform + 50% v/v EtAc, toluol and trifluoroethanol.

In a preferred embodiment of the invention, the biodegradable polymer layer has a thickness of about 5 to about 1000 nm. In one of the more preferred embodiments of the invention, the polymer layer has a thickness of about 100 to about 500 nm. In a further preferred embodiment, the polymer layer has a thickness of about 250 nm.

The whole setup of the invention is configured such that a change in the thickness of the polymer layer leads to a change of the colour of the device which is visible to the human eye. The expression "whole setup" comprises for this reason all three layers of the device, their material, their distances, the thickness of each layer and so on.

The thicknesses mentioned in the context of the reflecting layer, the nanoparticle layer and the polymer layer thus reflect certain embodiments of the invention. They can also be in a different range as long as the optical setup works, wherein a change in the thickness of the polymer layer leads to a change of the colour of the device which is visible to the human eye.

The change of the colour of the device according to the invention which is visible to the human eye is, therefore, due to the optical setup of the device and a change in the thickness of the polymer layer and not due to a change of colour of the polymer layer itself.

In yet another preferred embodiment of the invention, the device comprises an additional carrier layer. This carrier layer may be positioned on the reflecting layer. It may also be positioned on the nanoparticle layer. In both cases, the carrier layer is positioned on the opposite side of the polymer layer. In certain embodiments, this carrier layer is used to fix the device in the packaging of the natural product. In other preferred embodiments, the carrier layer is useful for the production of the device as set out below. In yet other preferred embodiments, the carrier layer is inert to any reaction with the natural product and allows biomolecules to penetrate to the next layer. The carrier layer may e.g. be in a preferred embodiment a PET-film, on top of which a gold layer is positioned as mirror layer, followed by the biodegradable polymer layer and the nanoparticle layer. In another embodiment, the carrier layer may e.g. be any standard transparent film, on top of which the nanoparticle layer is positioned, followed by the biodegradable polymer layer and the mirror layer. The carrier layer may be part of the preparation-process and may in one embodiment be of the same origin as the packaging material for the natural product. In other preferred embodiment, the additional carrier layer is an inorganic or organic carrier selected from the group comprising polyethylenterephtalate, glass, and the like.

In still another preferred embodiment of the invention, the device comprises a special type of carrier layer, namely a permeable layer having protective properties. Such a layer may be a hydrogel layer. This hydrogel layer me be positioned on the reflecting layer and/or on the nanoparticle layer. In one embodiment, this layer may be in direct contact with the natural product and allow the penetration of biomolecules to the reflecting layer and/or the nanoparticle layer, resp. The hydrogel layer may act as protection layer for the device and may represent a diffusion layer. The hydrogel layer may preferably be a crosslinked and/or stabilized food-contact compatible polymer layer which is swelling in contact with water and ensures that water is attracted in proximity to the biodegradable layer. The crosslinked hydrogel layer may in a preferred embodiment be a poly-acrylic-acid (PAA) layer. The PAA may have been neutralized with KOH so that it does not change the pH in the microenvironment of the biodegradable layer.

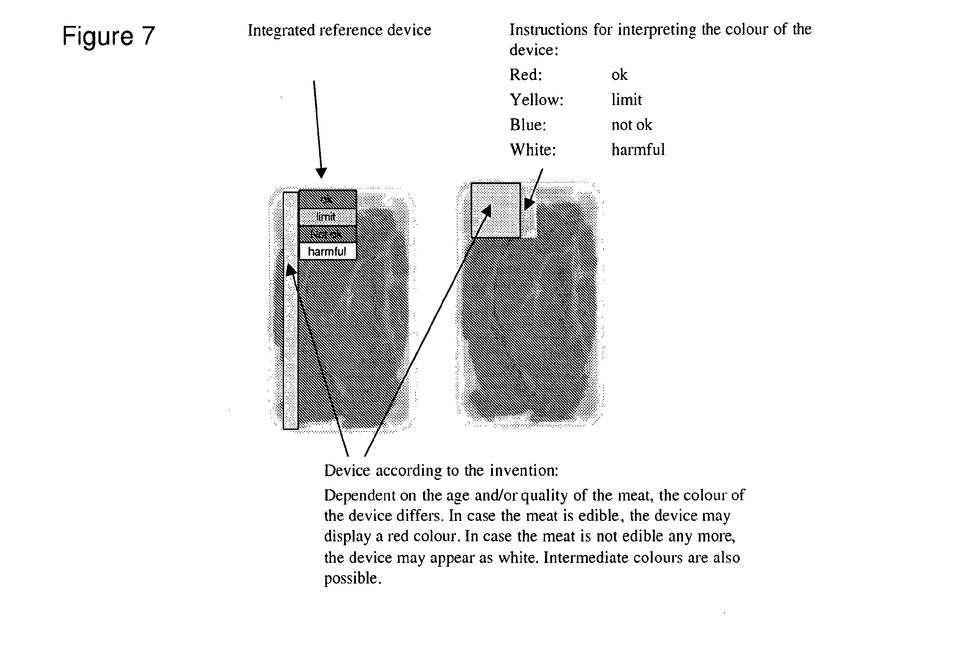

The device according to the invention may have different forms. In a preferred embodiment of the invention, the device may have the form of a square (figure 7). In a further preferred embodiment, the device may be a stripe. Both devices may be integrated into the packaging of e.g. meat (see figure 7). Both forms may have an identical surface area, but according to their form they cover different specific areas of e.g. meat. In case of the square, a certain area of meat is covered to a very good extent, whereas in case of a stripe, a broad area of meat is covered to a very good extent. In both cases, this is meant to account for the possibility that the spoilage of meat is not necessarily a homogenous process and, therefore, may start at different areas and at different points of time. By having the form of a stripe reaching from one end of the packaging to the other end or as big square on one spot (see figure 7), the inventors try to account for this problem by covering different areas of meat. In other embodiments of the invention, the form of the device may be a circle, rectangle, ellipse or any other suitable form.

The term "reference device" according to the present invention defines a device of a specific colour or a colour range, wherein the colour/colour range is not subjected to a change of colour. To this aim, the reference device is in a preferred embodiment of the invention coloured by any technique known to the person skilled in the art. In another embodiment of the invention, the reference device comprises a polymer layer which is not degradable by biomolecules as mentioned above and therefore does not change its thickness in case it is contacted by said biomolecules . Accordingly, the colour of the reference device after exposure to such biomolecules does not change. Thus, the reference device may have one colour which is identical to the colour of the device according to the invention in case the biodegradable polymer layer is totally intact. Furthermore, the reference device may have a second colour, which is identical to the device according to the invention in case the biodegradable polymer layer is substantially up to totally degraded by biomolecules as mentioned above. In this embodiment, the consumer is able to compare the colour of the device according to the invention to two possible thickness-conditions of the polymer layer of the invention. Of course, the reference device may comprise more than one or two colours for comparison reasons. In an also preferred embodiment, the reference device does not display certain specific colours, but is comprised of a non-degradable polymer layer ranging from the thickness of the device of the invention before any degradation to zero and, therefore, displays a colour range. Again, the consumer may compare the colour of the device according to this invention to said colour range. The reference device may be positioned directly next to the device of the invention. Furthermore, the reference device may inert and, therefore, may not influence the natural product itself.

Furthermore, in other embodiments of the present invention, methods for preparing the aforementioned devices are disclosed. In one embodiment, a reflecting layer, e.g. a thin gold layer is provided. In an alternative embodiment, Inconnel (by CPFilms, brand name: Lummalloy) is provided as said reflecting layer. Onto said reflecting layer, a biodegradable polymer layer, e.g. PLA, is applied. Onto said biodegradable polymer layer, a nanoparticle layer, e.g. a island layer made of gold, is applied. In another preferred embodiment, the reflecting layer is applied onto a carrier layer, already mentioned above, e.g. a PET-film is coated with a thin gold layer. In anther embodiment of the invention, a second carrier layer may be applied onto the nanoparticle layer. This leads in a preferred aspect of the invention to the following layers:
- carrier layer
- reflecting layer
- biodegradable polymer layer
- nanoparticle layer
- optionally a second carrier layer (e.g. a hydrogel layer)

In a further preferred embodiment of the invention, a carrier layer is provided, e.g. a translucent film, such as a PET film. Onto said carrier layer, a nanoparticle layer is applied, e.g. said gold-island layer. Onto said nanoparticel layer, a biodegradable polymer layer, e.g. PLA, is applied. Onto said biodegradable polymer layer, a reflecting layer, e.g. made of gold, is applied. A second carrier layer may be applied as well onto the reflecting layer on the opposite side of the biodegradable polymer layer. This leads in another preferred aspect of the invention to the following layers:
- carrier layer
- nanoparticle layer
- biodegradable polymer layer
- reflecting layer
- optionally a second carrier layer (e.g. a hydrogel layer)

As can be seen from the layers, the device prepared according to the first aspect may not necessarily differ from the device prepared according to the second aspect, although their methods for preparation clearly differ from each other. According to the use of the device as set out below, there may be certain advantages for either method of preparing the device.

In preferred methods for preparing such devices, the polymer layer may be applied by dip coating or film-printing techniques, such as gravure printing, or by spin coating. Such techniques are routine methods to the skilled person in the art. Any other technique known to the person skilled in the art leading to the application of thin polymer layers onto other layers may also be used. In preferred embodiments, PLA is used as material for the polymer layer. In the methods for preparing the polymer layer, PLA may be used in a concentration (weight/volume) ranging from about 1,5% w/v to about 20% w/v in a suitable solvent, such as Chloroform, trifluoroethanole, Toluole and the like. The concentration of the cross-linking agent used is also critical for the method of preparing the polymer layer. Desmodur might be used as cross-linking agent in a concentration (volume/volume) ranging from about 0,05% v/v to about 5,0% v/v. In a preferred aspect of the invention, the polymer layer is prepared with about 10% (w/v) PLA in triflouroethanole and about 2,0%(v/v) Desmodur as cross-linking agent by gravure printing.

In preferred methods for preparing such devices, the nanoparticle layer may be applied by sputter-coating, by evaporation or by chemical reactions, such as a chemical reaction using the reduction of HAuCl₄. In one preferred embodiment, the nanoparticle layer is made by sputter-coating Au onto a carrier such as a pretreated or nontreated PET film. Such techniques are routine methods to the skilled person in the art. Any other technique known to the person skilled in the art leading to the application of thin nanoparticle layers onto other layers may also be used.

In preferred methods for preparing such devices, the reflecting layer may be applied by vacuumcoating technologies such as evaporation or sputter coating or chemical reactions, such as direct application of gold nanoparticles through a chemical reaction using the reduction of HAuCl₄. In one preferred embodiment, the reflecting layer is made by sputter coating Inconnel onto a carrier such as a pretreated or nontreated PET film. Such techniques are routine methods to the skilled person in the art. Any other technique known to the person skilled in the art leading to the application of thin reflecting layers onto other layers may also be used.

In certain embodiments of the invention, a method for preparing a device comprising an additional hydrogel layer as set out above is provided. In the direct setup, the hydrogel layer is applied directly on top of the nanoparticle layer on the opposite side of the biodegradable polymer layer, such that the hydrogel layer may be in direct contact with the natural product. In the indirect setup, the hydrogel layer is applied directly on top of the reflecting layer on the opposite side of the biodegradable polymer layer, such that the hydrogel layer may be in direct contact with the natural product. The crosslinked hydrogel layer in a preferred embodiment is a Poly-Acrylic-Acid (PAA) Layer which has been applied by thin-film printing.

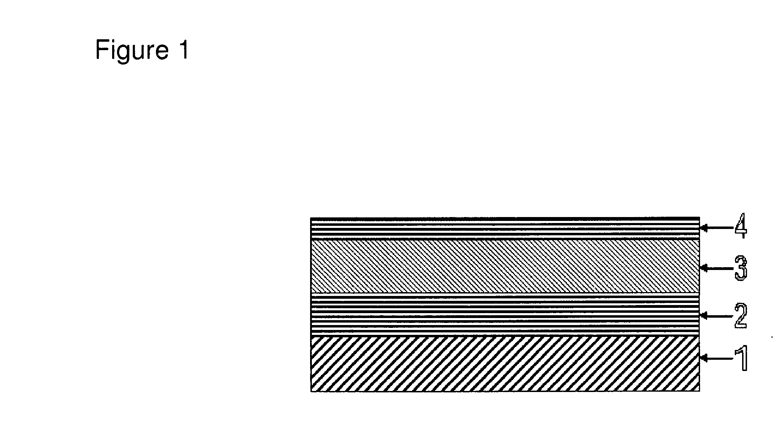

The sensor may thus be produced according to the invention by application of a nanometric layer of a biologically degradable polymer, e.g. poly-lactic-acid (PLA) onto a metalized carrier. A 1 to 30 nm thin gold layer is applied by sputtering onto the between 50 and 1000 nm thick polymer layer. A visible colouration of the surface is generated by the distance between reflector and mirror layer defined by the polymer. This colouration disappears or changes by the degradation of the polymer layer. Next to the colour formed by interference, a reference with a conventional dye or colours based on pigments can be printed in order to have a reference for the costumer when looking at the sensor. A schematic sensor is represented in figure 1.

In still other embodiments of the invention, methods for analyzing the age and/or quality of a natural product are provided. In one aspect of the invention, the natural product is directly contacted with the device such that the following set up of layers is present:
- carrier layer, e.g. PET film
- nanoparticle layer, e.g. Au
- polymer layer, e.g. PLA
- reflecting layer, e.g. Au.

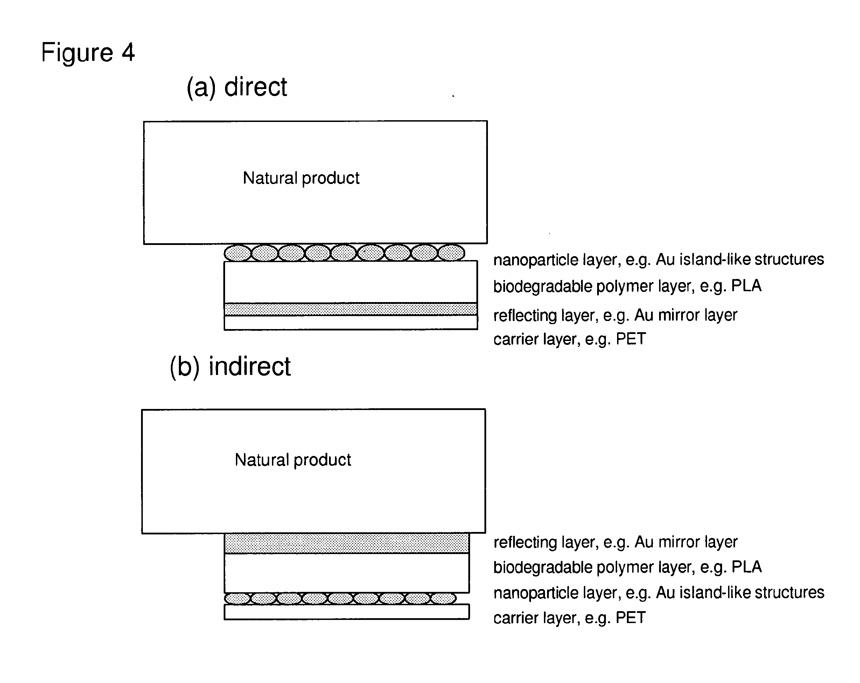

The reflecting layer contacts the natural product, e.g. meat. In this setup, the biomolecules as defined above are able to penetrate the reflecting layer and contact the polymer layer. The consumer is looking onto the carrier layer and, therefore, can directly see the colour of the device. The carrier layer may be part of the packaging of the natural product or may be a separate carrier, such as a PET-film. In this setup, the carrier layer, if present, is of any translucent material, which can be used with natural products, to allow the passage of incident and reflected light. Therefore, in this indirect setup, the consumer is able to analyze the colour of said device by directly looking at the device which may be integrated into the packaging of the natural product (see figure 4b).

In a second aspect of the invention, the natural product is directly contacted with the device such that the following set up of layers is present:
- nanoparticle layer, e.g. Au
- polymer layer, e.g. PLA
- reflecting layer, e.g. Inconnel
- carrier layer, e.g. glass

The natural product (e.g. fish) is contacted with the nanoparticle layer. In this setup, the biomolecules as defined above are able to penetrate the nanoparticle layer and contact the polymer layer. In order to analyze the colour of said device, the consumer may open the packaging to an extent to which the device is visible to the consumer, i.e. by separating the natural product from the device in order to look at the nanoparticle layer of the device. Again, the carrier layer may be part of the packaging of the natural product or may be a separate carrier, such as a PET-film. In this setup, the carrier layer, if present, can be of any material as there is no need for the carrier layer to be translucent. Therefore, in this direct setup, the consumer is able to analyze the colour of said device by partly opening up the packaging and looking at the device which may be integrated into the packaging of the natural product via said carrier layer (see figure 4a).

As set out above, there are different techniques of preparing said device. According to the setup used, either indirect or direct, the method for preparing the device may be chosen. In the indirect setup, the method used may in a preferred embodiment be to first sputter-coat Au onto a translucent PET film and then apply the PLA-polymer layer followed by the Au-reflecting layer. Said device may then be integrated into a translucent PET-film used for packaging of the natural product, e.g. meat, and positioned into said PET-film such that the reflecting layer is in direct contact with the meat and the translucent PET-film is directed towards the outside of the packaging.

The method for analyzing the age and/or quality of a natural product may comprise the comparison of the colour of the device to the colour of a reference device as defined above. The reference device may have one, two or several fixed colours corresponding to possible colours of the device of the invention according to possible thicknesses of the polymer layer as already set out above. In case the polymer layer is totally degraded, the device might be e.g. of white colour instead of a e.g. blue colour in case the polymer layer is not affected at all. The reference device may in this case be comprised of a white and a blue colour and, therefore, the consumer is able to compare the colour of the device to the reference-device and determine the age and/or quality of a natural product. If the device according to the present invention corresponds to the white colour of said reference device, the natural product is not intact any more and should not be used according to its purpose. On the other hand, if the device according to the invention corresponds to the blue colour of said reference device, the age and/or quality of said natural product is a condition, in which the natural product may be used according to its purpose. As set out before the reference device may display a colour range. In this case, the consumer can compare the colour of the device according to the invention to the colour range of the reference device and determine the age and/or quality of the natural product accordingly.

In other embodiments, the present invention is concerned with the use of a device according to the present invention for the analysis of the age and/or quality of a natural product. Via the degradation of a biodegradable polymer layer by enzymes and/or catabolic metabolites of the natural product itself or any associated organism, said age and/or quality can be analyzed. The higher the concentration of said biomolecules, the thinner the polymer layer of said device. Therefore, there is a correlation between the presence of e.g. enzymes of microorganisms responsible for spoiling foods and the thickness of the polymer layer. The thickness in turn correlates with the colour of the device visible to the human eye. Therefore, ultimately, the presence of e.g. microorganisms responsible for spoiling foods correlates with the colour of said device. Thus, the device may be used for analyzing the age and/or quality of a natural product. In case foods are analyzed, the edibility of such foods may be analyzed by the consumer.

The sensor may thus be used for the detection of specific reactions of degradation that occur during the aging of e.g. foods or cosmetic products. Such aging processes are often associated with e.g. the spoilage of foods.

Surprisingly, it thus has been found that a biologically degradable polymer is translating the activity of enzymes into visible signals in an optical interference thin layer setup. The decay of the thin layers leads to defmed changes in the colour of the surface. From the literature, such underlying degradation conditions are known, but could thus far only be visualized in the laboratory. While there is ultimately no guarantee on this theory, one assumes based on the present data that the enzymes penetrate into the optical thin layer setup and attack the bonds between the polymer-units. It has been observed that the stability of the polymer layer gets reduced and the layer brakes down or disintegrates and the cohesion of the optical thin layer systems breaks down.

Further preferred embodiments:
1. Sensor characterized in that a metallic mirror is applied onto a carrier, onto which a 50 to 1000 nm thick biodegradable polymer layer is applied, onto which a layer of metallic islands is applied in such a way that enzymes can diffuse to the polymer layer.
2. Sensor according to (1) characterized in that the interference colour is combined with a pigment or a dye based on a colour similar to the human eye.
3. Sensor according to (1)-(2) characterized in that a bifunctional radical crosslinking agent is used as a crosslinking agent.
4. Sensor according to (1)-(3), wherein diisocyanate is used as a crosslinking agent.
5. Sensor according to (1), characterized in that a hydrogel layer is applied onto the layer of metallic islands.
6. Method for preparing a sensor, wherein a metallic mirror is applied onto a carrier, and wherein a 50-1,000 nm thick biodegradable polymer layer is applied onto said mirror, and a layer of metallic islands is applied to said polymer layer in such a way that enzymes can diffuse to the polymer layer.
7. Method for preparing a sensor according to (6), wherein the polymer layer is prepared by dip coating.
8. Method for preparing a sensor according to (6), wherein the polymer layer is prepared by film printing.
9. Methods for preparing a sensor according to (6)-(8), wherein the polymer layer is stabilized by a chemical crosslinking agent.
10. Methods for preparing a sensor according to (6)-(9), wherein the active area of the sensor is combined with a regular colour of a printed reference.
11. Method for preparing a sensor according to (6)-(10), wherein the active sensory area is totally or partially coated with a hydrogel.
12. Use of a sensor according to (1)-(11), wherein the change of the colour of the surface by the action of enzymes is detected with the naked eye.
13. Use of a sensor according to (12), wherein it is possible for a user to detect small changes of the surface of the sensor by the comparison with the reference.
14. Use of a sensor according to (12), wherein a semi-quantitative detection of the amount of analyte is possible for the user by comparing the surface of the sensor with a reference scale.

A freshness sensor integrated into the packaging wherein a metallic mirror is applied onto a carrier, onto which a 50 to 1000 nm thick biodegradable polymer layer is applied, onto which a layer of metallic islands is applied such that enzymes are able to diffuse to the polymer layer.

The invention is further illustrated by the following examples, which should not be construed as limiting.

### EXAMPLES

### Example 1: Sensor for detecting the esterase concentration from bacillus sp.

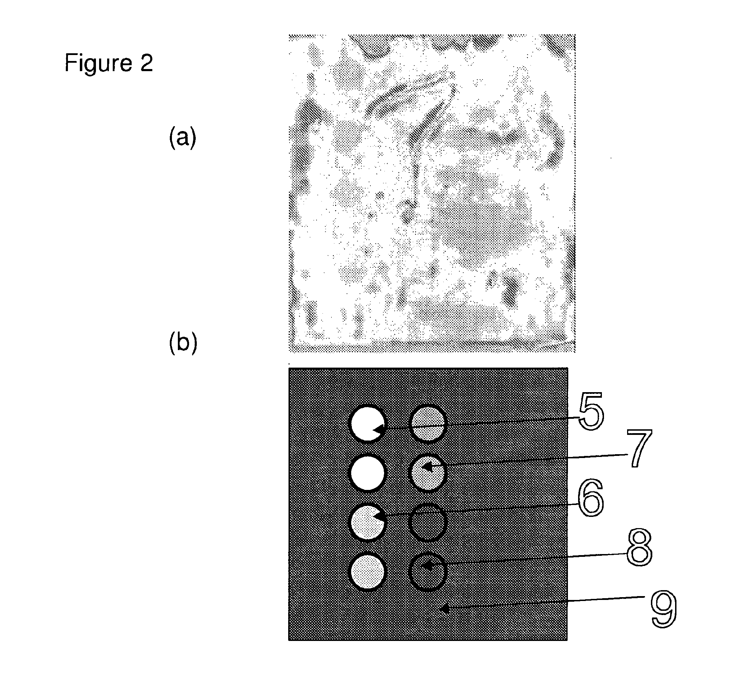

For this purpose, a 50 mm thick inorganic or organic carrier such as, for example, polyethylene terephtalate carrier or glass coated with 30 nm of gold is drawn with 6 cm/min out of a solution of 3 % (by weight) PLA (Biomer, MW 200,000 Da) and 0.5 % Desmodur (Jäckle Chemie) in chloroform. A tension-free film with a mass thickness of 120 nm is applied onto the mirror layer by dip coating. 4 nm of gold are applied to this nanometric thin polymer layer in such a way that a film of islands is formed on the surface. The resulting sensor shows a change of the surface colour within a short time frame if different concentrations of esterase act on it. Figure 2 shows a picture of a sensor platelet that has been incubated with different concentrations of an esterase solution.

In a further possible embodiment according to example 1, the following protocol was used: a polyethylene terephtalate carrier coated with 30 nm of gold was used. A tension-free polymer layer (200 nm) was applied onto the gold layer by dip coating. 6.5% PLA was used as biodegradable polymer with a linking agent Desmodur (3%) and in chloroform as solvent. Onto this layer, the nanoparticle layer was applied by sputter coating of Au to form a 5 nm thick Au island-structure. After preparation of such a device, different spots of the device were incubated with esterase-solutions of indicated concentrations (including a buffer control) for 4 h at 37°C.

The esterase-solution was prepared by dissolving esterase in 0.1 M Tris-HCl pH 8.2 to prepare a stock solution of 10 mg/ml. Corresponding dilutions were prepared in 0.1 M Tris-HCl pH 8.2.

### Example 2: Sensor for detecting a contamination in fish:

The sensor described in example 1 is placed as an insertion platelet underneath the fish in the conservation packaging. In doing so, the surface of the sensor is directly exposed to the liquids of the fish. The colour of the surface changes continuously for three days at room temperature from deep blue to milky white, which indicates the progressive decomposition of the fish.

### Example 3: Sensor for detecting molds on bread:

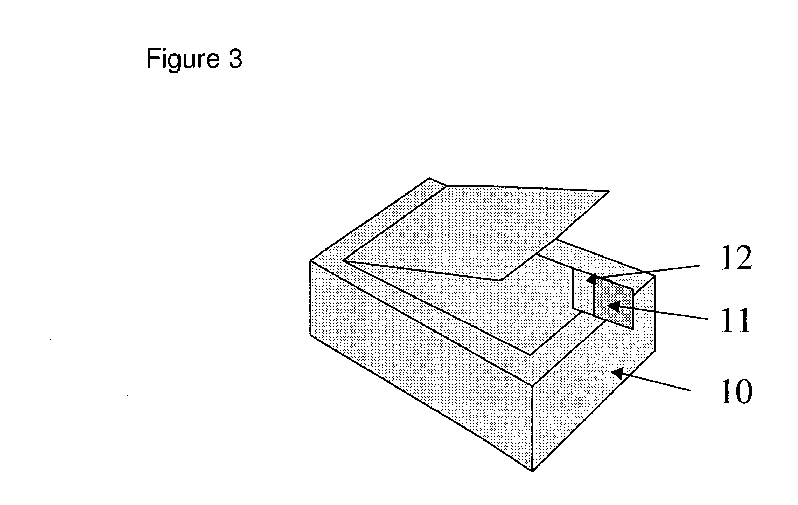

The sensor described in example 1 is coated with a hydrophilic hydrogel and integrated into the packaging of the bread as an adhesive label. The consumer may keep the marked area of the packaging in close proximity to the bread so that the mold growing on the bread also grows on the hydrogel and the enzymes secreted by the mold change the colour of the surface within a few days at room temperature from deep blue to a milky discoloration. Figure 3 shows the schematic application of the packaging with an integrated indicator.

In this specific example, the hydrogel was applied by dip coating.

### Example 4: Incubation of a device according to the invention with meat juice (direct setup)

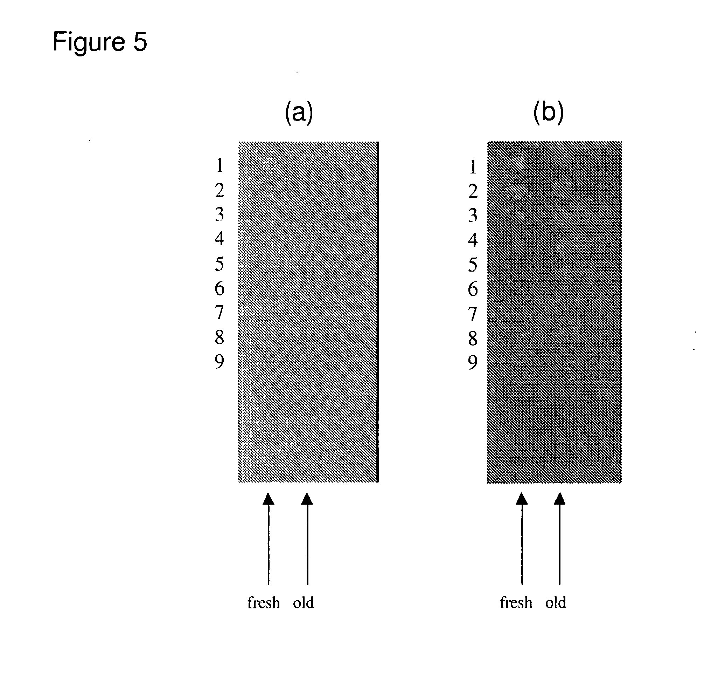

Inconnel (commercially available from CPFilms: brand name Lummalloy) was used as reflecting layer on top of PET film as carrier-layer. Onto the metallic reflector Inconnel, the polymer layer was applied by dip coating. The biodegradable polymer layer consisted of: 6.5% PLA (w/v), 3% Desmodur in Trifluorethanol. The thickness was about 200 nm. Onto the polymer layer, a nanoparticle layer was applied. This was done by sputter coating Au onto the polymer layer, resulting in a thickness of this Au-island-structure layer of about 5 nm. Either fresh or old meat juice homogenate as indicated below was pipetted onto the nanoparticle layer (this corresponds to the natural product contacting the reflecting layer and, therefore, to the direct setup) in different concentrations as indicated below. After an incubation of 4 h at 37 °C, the device was washed with ddH₂O, dried under an airstream and scanned as seen from the nanoparticle layer in two different ways as indicated in the figure legend to make the colours and degradation visible (figure 5 a and b).

### Pipetting scheme:

| | Fresh meat juice homogenate | Old meat juice homogenate |
|---|---|---|
| 1 | undiluted | undiluted |
| 2 | 1:2 | 1:2 |
| 3 | 1:4 | 1:4 |
| 4 | 1:8 | 1:8 |
| 5 | 1:16 | 1:16 |
| 6 | 1:32 | 1:32 |
| 7 | 1:64 | 1:64 |
| 8 | Buffer | Buffer |
| 9 | ddH₂O | ddH₂O |

Fresh and old meat juice homogenate was prepared according to the following protocol: The meat (bought as packaged meat in a supermarket) was homogenised in 20 g portions with 180 ml of a standard peptone-glycerol-buffer (also referred to in the following as buffer). Following the homogenisation, the extract was frozen in aliquots of 150 µl at -80°C resulting in fresh meat juice homogenate. For the preparation of old juice homogenate, the meat was incubated prior to the homogenization for a period of time that the expiration date was expired for at least 1 day at 4°C (in a refrigerator) to give spoiled meat. Otherwise it was treated in exactly the same way as described for the fresh meat juice homogenate.

Clearly, the incubation with the meat juice homogenate results in significant changes of the device in terms of its colour (figure 5a) as well as the degradation (figure 5b).

### Example 5: Incubation of a device according to the invention with meat juice (indirect setup)

Gold was used as reflecting layer and PET- film as carrier-layer. Onto the PET film as carrier layer the nanopartcile layer was applied by sputter coating with a gold, target for 30 sec at 0.08 mbar, then the polymer layer was applied by dip coating.

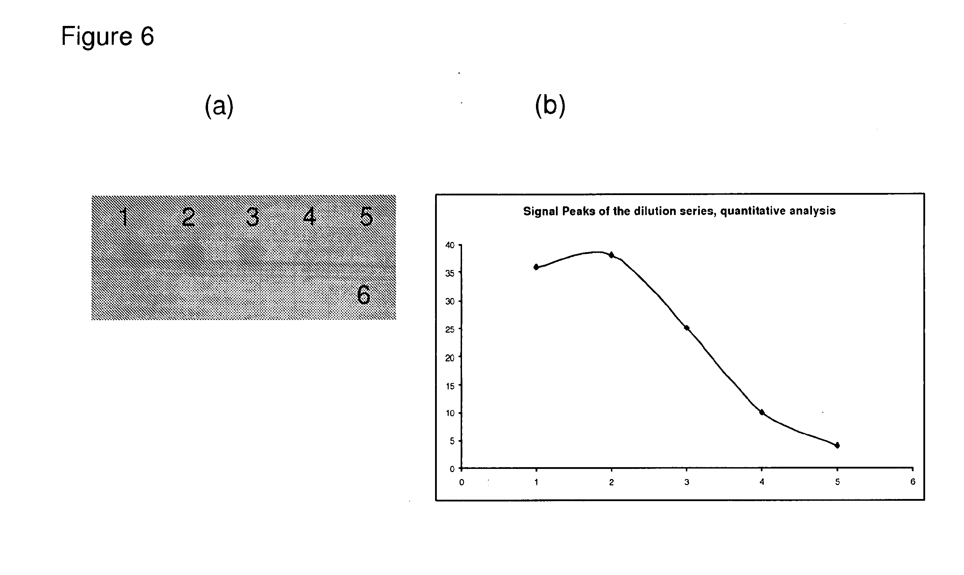

The biodegradable polymer layer consisted of: 6.5% PLA (w/v), 3% Desmodur in Trifluorethanol. The thickness was about 200 nm. Onto the polymer layer, a reflecting layer was applied. This was done by sputter coating with a gold, target for 120 sec at 0.08 mbar, resulting in a thickness of this Au-island-structure layer of about 30 nm. Old meat juice homogenate was pipetted onto the reflecting layer (this corresponds to the natural product contacting the nanoparticle layer and, therefore, to the indirect setup) in different concentrations as indicated below. After an incubation of 4 h at 37 °C, the device was washed with ddH₂O, dried under an airstream and scanned as seen from the nanoparticle layer (figure 6 a).

### Pipetting scheme:

| | meat juice homogenate |
|---|---|
| 1 | 1:1 |
| 2 | 1:2 |
| 3 | 1:3 |
| 4 | 1:4 |
| 5 | 1:5 |
| 6 | buffer |

To quantify the signals resulting from the incubation with meat juice of different concentrations, the intensities of the corresponding dots 1 to 6 were determined using standard methods and corresponding software. The result is shown in figure 6 b. Clearly, the curve correlates with the different dilutions of the meat juice homogenate. Therefore, the intensity (colour change) correlates with the amount of enzymes and catabolic metabolites present in the meat juice homogenate.

### Example 6: Exemplary use of a device according to the invention

In this example, the use of a device according to the invention is described for the analysis of the age and/or quality of packaged meat. Figure 7 illustrates two possible ways (which are, of course, not the only possibilities and thus not limiting): In the left picture, the device according to the invention is placed as stripe, in the right picture as square onto the natural product. In both cases, the device is integrated into the packaging and directly contacts the meat. Also in both cases, an indirect setup (for the setup of the layers see figure 4) is used. This means, that the consumer can assess the colour of the device directly from above without opening the packaging. This is possible because of the translucent packaging of the meat, in this case a PET-film. In the left case, an integrated reference device is also depicted. This reference device is comprised of four different colours corresponding to four different quality-conditions of the meat, which would be signalled by the colour of the device according to the invention: red for "ok", yellow for "limit", blue for "not ok" and white for "harmful". According to the quality and/or age of the meat, the device according to the invention changes its colour (not shown in figure 7). In case the meat is edible and of good quality, the colour of the device would thus appear red. In case the meat is spoiled and not edible any more, the colour of the device would thus appear white. Of course, different colours in between are also possible. By comparing the colour of the device according to the invention to the reference device, the consumer is able to directly analyze the quality of the product. In case there is no integrated reference device (right picture), the description depicted next to the device according to the invention contains instructions for interpreting the colour of the device.

## Claims

1. A device comprising
a) a reflecting layer (2)
b) a nanoparticle layer
c) a biodegradable polymer layer (3) positioned between said reflecting layer and said nanoparticle layer, wherein said biodegradable polymer layer is selected from the group of polymers comprising polylactic acid (PLA), poly-L-lactic acid (PLLA), PLGA, PHB and polyvinylcaprolactame (PVCL);
wherein the device is configured in such a way that biomolecules capable of degrading said polymer layer are able to penetrate said reflecting layer and/or said nanoparticle layer in order to contact said polymer layer and wherein the device is configured in such a way that a change in the thickness of said polymer layer results in a colour change visible to to the human eye.

2. A device according to claim 1 wherein said reflecting layer and/or said nanoparticle layer have a thickness of 1 to 100 nm and said biodegradable polymer layer has a thickness of 5 to 1000 nm.

3. A device according to claims 1 and 2 wherein said reflecting layer comprises a mirror layer made of a metal.

4. A device according to claim 3 wherein said mirror layer is made of gold.

5. A device according to claim 4 wherein said mirror layer made of gold has a thickness of 10 to 30 nm.

6. A device according to any of claims 1 to 5 wherein said nanoparticle layer comprises a plurality of island-like structures wherein said islands have a size of 5 to 50 nm in diameter.

7. A device according to claim 6 wherein said nanoparticle layer is made of a metal.

8. A device according to claim 7 wherein said nanoparticle layer is made of gold.

9. A device according to claim 8 wherein said nanoparticle layer made of gold consisting essentially of a plurality of island-like structures wherein said islands have a size of 5 to 50 nm in diameter, has a thickness of 2 to 20 nm.

10. A device according to any of claims 1 to 9 wherein said biodegradable polymer layer is degradable by biomolecules comprising enzymes and/or catabolic metabolites.

11. A device according to claim 10 wherein said biodegradable polymer layer has a thickness of 100 to 500 nm.

12. A device according to any of claims I to 11 comprising an additional carrier layer (1) on the reflecting layer and/or the nanoparticle layer, in each case being positioned on the side of the reflecting and/or the nanoparticle layer opposite of said polymer layer.

13. A device according to any of claims 1. to 12 wherein the device also comprises a reference device.

14. A device according to any of claims 1 to 13 comprising
a) a mirror layer made of gold with a thickness of 10 to 30 nm
b) a nanoparticle layer made of gold comprising a plurality of islands wherein said islands have a size of 5 to 50 nm in diameter with a thickness of 2 to 20 nm
c) a biodegradable polymer layer with a thickness of 100 to 500 nm positioned between said reflecting layer and said nanoparticle layer
d) a reference device.

15. A method for preparing a device according to any of claims 1 to 14 wherein said method comprises the steps of
a) Providing a reflecting layer (2)
b) Applying a biodegradable polymer layer (3) onto said reflecting layer
c) Applying a nanoparticle layer onto said polymer layer.

16. A method according to claim 15 wherein said method comprises the additional step of providing a carrier and applying said reflecting layer onto said carrier.

17. A method for preparing a device according to any of claims 12 to 14 wherein said method comprises the steps of
a) Providing a carrier (1)
b) Applying a nanoparticle layer onto said carrier
c) Applying a biodegradable polymer layer (3) onto said nanoparticle layer
d) Applying a reflecting layer (2) onto said biodegradable polymer layer.

18. A method according to any of claims 15 to 17 wherein the polymer layer is applied by dip coating or film-printing.

19. A method according to any of claims 1 to 18 wherein the nanoparticle layer and/or the reflecting layer is applied by sputter-coating, by evaporation or by chemical reactions.

20. The use of a device according to any of claims 1 to 14 for the analysis of the age and/or quality of a natural product comprising foods and cosmetical products.

21. The use of a device according to claim 20 for the analysis of the age and/or quality of a natural product by detecting microorganisms present in the natural product.

22. The use of a device according to claim 21 for the analysis of the age and/or quality of a natural product by detecting enzymes of microorganisms and/or of the natural product and/or catabolic metabolites via the degradation of said biodegradable polymer by said enzymes and/or catabolic metabolites.

23. The use of a device according to claim 20 for the analysis of the age and/or quality of a natural product comprising foods and cosmetical products comprising the following steps:
a) Providing a device according to any of claims 1 to 14
b) Contacting said device with said natural product
c) Determining the colour of said device
d) Comparing the colour of said device with a reference device
e) Determining the age and/or quality of said natural product according to this comparison.

24. The use of a device according to claim 23 for the analysis of the age and/or quality of a natural product wherein the reflecting layer or the nanoparticle layer of said device is being contacted in step b) with said natural product in such a way that biomolecules are able to penetrate the reflecting layer or the nanoparticle layer and contact the polymer layer.

## Patentansprüche

1. Eine Vorrichtung umfassend
a) eine reflektierende Schicht (2)
b) eine Nano-Partikelschicht
c) eine bioabbaubare Polymerschicht (3), die zwischen besagter reflektierender Schicht und besagter Nano-Partikelschicht platziert ist, wobei besagte bioabbaubare Polymerschicht ausgewählt ist aus der Gruppe von Polymeren umfassend Polymilchsäure (PLA), Poly-L-Milchsäure (PLLA), PLGA, PHB und Polyvinylcaprolactam (PVCL);
wobei die Vorrichtung so konfiguriert ist, dass Biomoleküle, die dazu in der Lage sind, die besagte Polymerschicht abzubauen, die besagte reflektierende Schicht und/oder die besagte Nano-Partikelschicht penetrieren können, um besagte Polymerschicht zu kontaktieren und wobei die Vorrichtung so konfiguriert ist, dass eine Veränderung in der Dicke der besagten Polymerschicht zu einer Farbänderung führt, die für das menschliche Auge sichtbar ist.

2. Eine Vorrichtung gemäß Anspruch 1, wobei besagte reflektierende Schicht und/oder besagte Nano-Partikelschicht eine Dicke von 1 bis 100 nm haben und besagte bioabbaubare Polymerschicht eine Dicke von 5 bis 1000 nm hat.

3. Eine Vorrichtung gemäß den Ansprüchen 1 und 2, wobei besagte reflektierende Schicht eine Spiegelschicht aus Metall umfasst.

4. Eine Vorrichtung gemäß Anspruch 3, wobei besagte Spiegelschicht aus Gold ist.

5. Eine Vorrichtung gemäß Anspruch 4, wobei besagte Spiegelschicht aus Gold eine Dicke von 10 bis 30 nm hat.

6. Eine Vorrichtung gemäß einem der Ansprüche 1 bis 5, wobei besagte Nano-Partikelschicht eine Mehrzahl von Insel-ähnlichen Strukturen umfasst, wobei besagte Inseln eine Größe von 5 bis 50 nm im Durchmesser besitzen.

7. Eine Vorrichtung gemäß Anspruch 6, wobei besagte Nano-Partikelschicht aus Metall ist.

8. Eine Vorrichtung gemäß Anspruch 7, wobei besagte Nano-Partikelschicht aus Gold ist.

9. Eine Vorrichtung gemäß Anspruch 8, wobei besagte Nano-Partikelschicht aus Gold, die im Wesentlichen aus einer Mehrzahl von Insel-ähnlichen Strukturen besteht, wobei besagte Insel eine Größe von 5 bis 50 nm im Durchmesser haben, eine Dicke von 2 bis 20 nm besitzt.

10. Eine Vorrichtung gemäß einem der Ansprüche 1 bis 9, wobei besagte bioabbaubare Polymerschicht abbaubar ist durch Biomoleküle umfassend Enzyme und/oder katabolische Metabolite.

11. Eine Vorrichtung gemäß Anspruch 10, wobei besagte bioabbaubare Polymerschicht eine Dicke von 100 bis 500 nm hat.

12. Eine Vorrichtung gemäß einem der Ansprüche 1 bis 11, umfassend eine zusätzliche Trägerschicht (1) auf der reflektierenden Schicht und/oder der Nano-Partikelschicht, wobei diese in jedem Fall auf der Seite der reflektierenden und/oder der Nano-Partikelschicht, die gegenüber von besagter Polymerschicht liegt, platziert ist.

13. Eine Vorrichtung gemäß einem der Ansprüche 1 bis 12, wobei die Vorrichtung auch eine Referenz-Vorrichtung umfasst.

14. Eine Vorrichtung gemäß einem der Ansprüche 1 bis 13 umfassend
a) eine Spiegelschicht aus Gold mit einer Dicke von 10 bis 30 nm
b) eine Nano-Partikelschicht aus Gold umfassend eine Mehrzahl von Inseln, wobei besagte Inseln eine Größe von 5 bis 15 nm im Durchmesser haben, mit einer Dicke von 2 bis 20 nm
c) eine bioabbaubare Polymerschicht mit einer Dicke von 100 bis 500 nm, platziert zwischen besagter reflektierenden Schicht und besagter Nano-Partikelschicht
d) eine Referenz-Vorrichtung.

15. Eine Methode zur Herstellung einer Vorrichtung gemäß einem der Ansprüche 1 bis 14, wobei besagte Methode die folgenden Schritte umfasst
a) Zurverfügungstellen einer reflektierenden Schicht (2)
b) Aufbringen einer bioabbaubaren Polymer-Schicht (3) auf besagte reflektierende Schicht
c) Aufbringen einer Nano-Partikelschicht auf besagte Polymerschicht.

16. Eine Methode gemäß Anspruch 15, wobei besagte Methode den zusätzlichen Schritt umfasst, eine Trägerschicht zur Verfügung zu stellen und besagte reflektierende Schicht auf besagte Trägerschicht aufzubringen.

17. Eine Methode zur Herstellung einer Vorrichtung gemäß einem der Ansprüche 12 bis 14, wobei besagte Methode die folgenden Schritte umfasst
a) Zurverfügungstellen einer Trägerschicht (1)
b) Aufbringen einer Nano-Partikelschicht auf besagte Trägerschicht
c) Aufbringen einer bioabbaubaren Polymerschicht (3) auf besagte Nano-Partikelschicht
d) Aufbringen einer reflektierenden Schicht (2) auf besagte bioabbaubare Polymerschicht.

18. Eine Methode gemäß einem der Ansprüche 15 bis 17, wobei die Polymerschicht durch Tauchbeschichtung oder Filmdruck aufgebracht wird.

19. Eine Methode gemäß einem der Ansprüche 15 bis 18, wobei die Nano-Partikelschicht und/oder die reflektierende Schicht durch Spritzbeschichtung, durch Verdampfen oder durch chemische Reaktionen aufgebracht wird.

20. Die Verwendung einer Vorrichtung gemäß einem der Ansprüche 1 bis 14 zur Analyse des Alters und/oder der Qualität eines natürlichen Produktes umfassend Lebensmittel und kosmetische Produkte.

21. Die Verwendung einer Vorrichtung gemäß Anspruch 20 zur Analyse des Alters und/oder der Qualität eines natürlichen Produktes durch die Detektion von Mikroorganismen, die in dem natürlichen Produkt vorhanden sind.

22. Die Verwendung einer Vorrichtung gemäß Anspruch 21 zur Analyse des Alters und/oder der Qualität eines natürlichen Produktes durch die Detektion von Enzymen von Mikroorganismen und/oder des natürlichen Produkts und/oder von katabolischen Metaboliten über den Abbau des besagten bioabbaubaren Polymers durch besagte Enzyme und/oder katabolische Metabolite.

23. Die Verwendung einer Vorrichtung gemäß Anspruch 20 zur Analyse des Alters und/oder der Qualität eines natürlichen Produktes umfassend Lebensmittel und kosmetische Produkte umfassend die folgenden Schritte:
a) Zurverfügungstellen einer Vorrichtung gemäß einem der Ansprüche 1 bis 14
b) Inkontaktbringen besagter Vorrichtung mit besagtem natürlichen Produkt
c) Bestimmung der Farbe der besagten Vorrichtung
d) Vergleich der Farbe von besagter Vorrichtung mit einer Referenz-Vorrichtung
e) Bestimmung des Alters und/oder der Qualität des besagten natürlichen Produkts gemäß dieses Vergleichs.

24. Die Verwendung einer Vorrichtung gemäß Anspruch 23 zur Analyse des Alters und/oder der Qualität eines natürlichen Produktes, wobei die reflektierende Schicht oder die Nano-Partikelschicht der besagten Vorrichtung in Schritt b) mit besagtem natürlichen Produkt in Kontakt gebracht wird, und zwar in einer solchen Art und Weise, dass Biomoleküle die reflektierende Schicht oder die Nano-Partikelschicht penetrieren und die Polymerschicht kontaktieren können.

## Revendications

1. Dispositif comprenant :
a) une couche réfléchissante (2) ;
b) une couche de nanoparticules ;
c) une couche de polymère biodégradable (3) positionnée entre ladite couche réfléchissante et ladite couche de nanoparticules, ladite couche de polymère biodégradable étant choisie dans le groupe de polymères comprenant l'acide polylactique (PLA), l'acide poly-L-lactique (PLLA), le PLGA, le PHB et le polyvinylcaprolactame (PVCL) ;
ledit dispositif étant configuré de telle sorte que des biomolécules capables de dégrader ladite couche de polymère soient capables de pénétrer dans ladite couche réfléchissante et/ou ladite couche de nanoparticules afin d'entrer en contact avec ladite couche de polymère, et ledit dispositif étant configuré de telle sorte qu'une variation de l'épaisseur de ladite couche de polymère ait pour résultat un changement de couleur visible à l'oeil humain.

2. Dispositif suivant la revendication 1, dans lequel ladite couche réfléchissante et/ou ladite couche de nanoparticules ont une épaisseur de 1 à 100 nm et ladite couche de polymère biodégradable a une épaisseur de 5 à 1000 nm.

3. Dispositif suivant les revendications 1 et 2, dans lequel ladite couche réfléchissante comprend une couche miroir constituée d'un métal.

4. Dispositif suivant la revendication 3, dans lequel ladite couche miroir est constituée d'or.

5. Dispositif suivant la revendication 4, dans lequel ladite couche miroir constituée d'or a une épaisseur de 10 à 30 nm.

6. Dispositif suivant l'une quelconque des revendications 1 à 5, dans lequel ladite couche de nanoparticules comprend une pluralité de structures en îlots dans lesquelles lesdits îlots ont un diamètre de 5 à 50 nm.

7. Dispositif suivant la revendication 6, dans lequel ladite couche de nanoparticules est constituée d'un métal.

8. Dispositif suivant la revendication 7, dans lequel ladite couche de nanoparticules est constituée d'or.

9. Dispositif suivant la revendication 8, dans lequel ladite couche de nanoparticules constituée d'or, consistant essentiellement en une pluralité de structures en îlots, dans lesquelles lesdits îlots ont un diamètre de 5 à 50 nm, a une épaisseur de 2 à 20 nm.

10. Dispositif suivant l'une quelconque des revendications 1 à 9, dans lequel ladite couche de polymère biodégradable est dégradable par des biomolécules comprenant des enzymes et/ou des métabolites cataboliques.

11. Dispositif suivant la revendication 10, dans lequel ladite couche de polymère biodégradable a une épaisseur de 100 à 500 nm.

12. Dispositif suivant l'une quelconque des revendications 1 à 11, comprenant une couche de support supplémentaire (1) sur la couche réfléchissante et/ou la couche de nanoparticules, dans chaque cas positionnée du côté de la couche réfléchissante et/ou couche de nanoparticules à l'opposé de ladite couche de polymère.

13. Dispositif suivant l'une quelconque des revendications 1 à 12, ledit dispositif comprenant également un dispositif de référence.

14. Dispositif suivant l'une quelconque des revendications 1 à 13 comprenant
a) une couche miroir constituée d'or ayant une épaisseur de 10 à 30 nm,
b) une couche de nanoparticules constituée d'or comprenant une pluralité d'îlots, dans laquelle lesdits îlots ont un diamètre de 5 à 50 nm, en une épaisseur de 2 à 20 nm,
c) une couche de polymère biodégradable ayant une épaisseur de 100 à 500 nm positionnée entre ladite couche réfléchissante et ladite couche de nanoparticules,
d) un dispositif de référence.

15. Procédé pour préparer un dispositif suivant l'une quelconque des revendications 1 à 14, ledit procédé comprenant les étapes consistant à
a) fournir une couche réfléchissante (2),
b) appliquer une couche de polymère biodégradable (3) sur ladite couche réfléchissante,
c) appliquer une couche de nanoparticules sur ladite couche de polymère.

16. Procédé suivant la revendication 15, ledit procédé comprenant l'étape supplémentaire consistant à fournir un support et à appliquer ladite couche réfléchissante sur ledit support.

17. Procédé pour préparer un dispositif suivant l'une quelconque des revendications 12 à 14, ledit procédé comprenant les étapes consistant à
a) fournir à un support (1),
b) appliquer une couche de nanoparticules sur ledit support,
c) appliquer une couche de polymère biodégradable (3) sur ladite couche de nanoparticules,
d) appliquer une couche réfléchissante (2) sur ladite couche de polymère biodégradable.

18. Procédé suivant l'une quelconque des revendications 15 à 17, dans lequel la couche de polymère est appliquée par revêtement par immersion ou impression d'un film.

19. Procédé suivant l'une quelconque des revendications 15 à 18, dans lequel la couche de nanoparticules et/ou la couche réfléchissante sont appliquées par revêtement par pulvérisation cathodique, par évaporation ou par des réactions chimiques.

20. Utilisation d'un dispositif suivant l'une quelconque des revendications 1 à 14, pour l'analyse de l'âge et/ou de la qualité d'un produit naturel comprenant des denrées alimentaires et des produits cosmétiques.

21. Utilisation d'un dispositif suivant la revendication 20, pour l'analyse de l'âge et/ou de la qualité d'un produit naturel en détectant des micro-organismes présents dans le produit naturel.

22. Utilisation d'un dispositif suivant la revendication 21, pour l'analyse de l'âge et/ou de la qualité d'un produit naturel en détectant les enzymes de micro-organismes et/ou du produit naturel et/ou de métabolites cataboliques par dégradation dudit polymère biodégradable par lesdites enzymes et/ou lesdits métabolites cataboliques.

23. Utilisation d'un dispositif suivant la revendication 20, pour l'analyse de l'âge et/ou de la qualité d'un produit naturel comprenant des denrées alimentaires et des produits cosmétiques, comprenant les étapes suivantes :
a) fourniture d'un dispositif suivant l'une quelconque des revendications 1 à 14,
b) mise en contact dudit dispositif avec ledit produit naturel,
c) détermination de la couleur dudit dispositif,
d) comparaison de la couleur dudit dispositif à celle d'un dispositif de référence,
e) détermination de l'âge et/ou de la qualité dudit produit naturel d'après cette comparaison.

24. Utilisation d'un dispositif suivant la revendication 23, pour l'analyse de l'âge et/ou de la qualité d'un produit naturel, dans laquelle la couche réfléchissante ou la couche de nanoparticules dudit dispositif est mise en contact dans l'étape b) avec ledit produit naturel de telle sorte que des biomolécules soient capables de pénétrer dans la couche réfléchissante ou la couche de nanoparticules et d'entrer en contact avec la couche de polymère.
